# EUROPEAN PATENT SPECIFICATION

(11) **EP 1 879 572 B1**
(45) Date of publication and mention of the grant of the patent: **04.05.2011**
(21) Application number: 06747719.0
(22) Date of filing: 10.05.2006
(51) Int. Cl.: A61K 31/404, A61K 31/4025, A61K 31/4545, A61K 31/495, A61K 31/452, A61K 31/501, A61K 31/4439, A61K 31/5513, A61K 31/4709, A61K 45/06, A61P 15/06, A61K 38/12

(54) **USE OF ANTAGONISTS OF OXYTOCIN AND/OR VASOPRESSIN IN ASSISTED REPRODUCTION**
VERWENDUNG VON ANTAGONISTEN VON OXYTOCIN UND/ODER VASOPRESSIN BEI DER ASSISTIERTEN FORTPFLANZUNG
UTILISATION D'ANTAGONISTES DE L'OXYTOCINE ET/OU DE LA VASOPRESSINE DANS LA REPRODUCTION ASSISTEE

(30) Priority: 10.05.2005 PL 37495405; 12.08.2005 PL 37660705; 04.11.2005 US 733916 P
(43) Date of publication of application: 23.01.2008
(73) Proprietor: Ferring B.V., 2132 JX Hoofddorp (NL)
(72) Inventor: KUCZYNSKI, Waldemar, PL-15-338 Bialystok (PL); PIERZYNSKI, Piotr, PL-15-776 Bialystok (PL)
(74) Representative: HOFFMANN EITLE
(86) International application number: PCT/PL2006/000029
(87) International publication number: WO 2006/121362

(56) References cited:
- WO-A-96/09824
- US-A- 5 962 413
- US-A1- 2001 056 068
- US-A1- 2004 059 132
- LESNY P ET AL: "The junctional zone of the uterus and its contractions" BJOG: AN INTERNATIONAL JOURNAL OF OBSTETRICS AND GYNAECOLOGY 2004 UNITED KINGDOM, vol. 111, no. 11, 2004, pages 1182-1189, XP002402302 ISSN: 1470-0328
- KERIN J F P ET AL: "A simple technique for human embryo transfer into the uterus" LANCET 1981 UNITED KINGDOM, vol. 2, no. 8249, 1981, pages 726-727, XP008069796
- GRIGSBY PETA L ET AL: "Inhibition of premature labor in sheep by a combined treatment of nimesulide, a prostaglandin synthase type 2 inhibitor, and atosiban, an oxytocin receptor antagonist" AMERICAN JOURNAL OF OBSTETRICS AND GYNECOLOGY, vol. 183, no. 3, September 2000 (2000-09), pages 649-657, XP002402303 ISSN: 0002-9378
- HOMEIDA A M ET AL: "INHIBITION OF LUTEAL FUNCTION BY OXYTOCIN ANTAGONIST IN GOATS CAPRA-HIRCUS" JOURNAL OF REPRODUCTION AND FERTILITY, vol. 94, no. 1, 1992, pages 279-285, XP008069839 ISSN: 0022-4251
- KEELAN J A ET AL: "The molecular mechanisms of term and preterm labor: Recent progress and clinical implications" CLINICAL OBSTETRICS AND GYNECOLOGY 1997 UNITED STATES, vol. 40, no. 3, 1997, pages 460-478, XP008069831 ISSN: 0009-9201
- BALES K L ET AL: "EFFECTS OF NEONATAL OXYTOCIN MANIPULATIONS ON MALE REPRODUCTIVE POTENTIAL IN PRAIRIE VOLES" PHYSIOLOGY AND BEHAVIOR, ELSEVIER SCIENCE LTD., OXFORD, GB, vol. 81, no. 3, May 2004 (2004-05), pages 519-526, XP008061774 ISSN: 0031-9384
- PIERZYNSKI P ET AL: "Inhibitory effect of barusiban and atosiban on oxytocin-induced contractions of myometrium from preterm and term pregnant women" JOURNAL OF THE SOCIETY FOR GYNECOLOGIC INVESTIGATION, ELSEVIER, NEW YORK, NY, US, vol. 11, no. 6, September 2004 (2004-09), pages 384-387, XP004550637 ISSN: 1071-5576
- FUCHS A-R ET AL: "OXYTOCIN ANTAGONIST Ä1-D(CH2)5,TYR(ME)2,THR4,TYR-NH29ÜORNITHIN E VASOTOCIN INHIBITS OXYTOCIN-INDUCED PROSTAGLANDIN F2.ALPHA. RELEASE IN LATE-PREGNANT COWS" BIOLOGY OF REPRODUCTION, SOCIETY FOR THE STUDY OF REPRODUCTION, CHAMPAIGN, IL, US, vol. 57, no. 2, 1997, pages 436-441, XP008061779 ISSN: 0006-3363
- VALENTIN L ET AL: "EFFECTS OF A VASOPRESSIN ANTAGONIST IN WOMEN WITH DYSMENORRHEA" GYNECOLOGIC AND OBSTETRIC INVESTIGATION, KARGER, BASEL, CH, vol. 50, no. 3, October 2000 (2000-10), pages 170-177, XP008061776 ISSN: 0378-7346

## Description

The present invention relates to the use of barusiban or its combinations with other substances for the manufacture of a medicament, which main profile of action is an inhibition of oxytocin and/or vasopressin receptors in non pregnant uteri of mammals, leading to improvement of uterine receptivity at embryo transfer. The invention further relates to the use of above mentioned substances for the manufacture of a medicament for regulation of uterine contractility in subjects undergoing the procedure of artificial insemination.

Techniques of assisted reproduction are applied in humans for the treatment of infertility and in animals for producing pregnancies. Infertility, which affects about 10% of human pairs worldwide, may be treated by in vitro fertilization and embryo transfer (IVF-ET) or in less complicated cases - by artificial insemination. Success rate of IVF-ET procedures in humans usually ranges between 10% and 40% pregnancies per cycle of treatment, for insemination a level of 20% may be reached. Generally, a success of an embryo transfer is dependant on uterine receptivity, an entity that is defined as an ability of uterus to provide optimal conditions mandating proper implantation and embryo development. Basic components of uterine receptivity are uterine contractile activity and the condition of endometrium. Exaggerated uterine contractility occurring during the embryo transfer may expel embryos from the uterus towards vagina or oviducts, which may be a cause of unsuccessful treatment, or - in latter case - a cause of extrauterine pregnancy - a serious, potentially life-threatening complication. Additionally, a success of artificial insemination - apart from the sperm quality - is also correlated to the intensity and direction of uterine contraction waves as well as to the condition of endometrium. When uterine contractions are directed from uterine fundus to the cervix, sperm injected into the uterus during the procedure is moved out of the uterus, which affects the efficacy of this procedure.

In humans, cycles, where intrauterine implantation occurs can be characterized with a decreased uterine contractile activity. Uterine contractions also influence embryo implantation in raising animals. It is known that implantation rate is negatively correlated to the frequency of uterine contractions. Difference in transfer success rates between women of high and low uterine contractile activity may exceed 50%. Additionally to that, uterine-derived prostaglandins decrease the perfusion of endometrium, impairing the uterine receptivity. Application of medicaments that decrease uterine contractility, such as beta agonists is connected to frequent adverse reactions and do not influence the transfer success rates.

Lesny et al., Intl. J. of Obstetrics and Gynaecology 2004, vol. 111, no. 11, pp. 1182-1189, teaches that uterine contractions are important in sperm transport and embryo implantation. Increased junctional zone contractions have been linked directly to the failure of embryo transfer. To overcome this, treatment with the oxytocin antagonist Atosiban was proposed.

Kerin et al., Lancet, 1981, vol. 2, no. 8249, pp 726-727, refers to a technique for human embryo transfer into the uterus. The authors suggest that treatment with specific oxytocin antagonists may overcome problems associated with increased uterine tone.

The aim of the invention is to provide medicaments that increase the success rate of assisted reproduction procedures and to identify the substances that could be used for production of such medicaments, considering that these medicaments should be free of side effects that characterize the ones currently used and be of improved clinical efficacy in assisted reproduction.

The matter of invention is a use which is defined in attached claims. In particular, it pertains to the application of barusiban for the manufacture of a medicament for improvement of uterine receptivity in embryo transfers or in artificial inseminations.

In detail, the invention relates to the use of the effective quantity of barusiban for manufacture of medicaments applied in the procedures of assisted reproduction that could be applied before, during and after the embryo transfer and that act by improving the uterine receptivity. According to the invention, Barusiban is administered enterally or parenterally in the 24h dose ranging from 0,01 mg up to 10g.

Particular use according to the invention relates to the treatment of infertility in humans, specifically to the procedure of embryo transfer, especially to the transfer of fresh or frozen/thawed embryos. Advantageous application according to the invention relates to the in vitro fertilization-embryo transfer procedure (IVF-ET), or relates to the embryo transfer, where oocyte or sperm - components of embryo are taken from the donor(s). The invention specifically relates to the treatment carried out in raising animals - cows, pigs, horses, sheep, where embryo transfer procedure is done. The implementation of the invention additionally relates to the application of other medicaments that could be applied in the assisted reproduction, especially to nitric oxide donors, substrates of the nitric oxide synthase, progestagens, prostaglandin antagonists, methyloxantines, beta-agonists, prostacyclin agonists.

Particularly, medicaments produced according to the defined invention may be used during the procedures of assisted reproduction, specifically for the regulation of uterine contractile activity, that improves the sperm transport in female genital tract after the artificial insemination. In specific implementation of the patent it relates to the treatment that is carried out in humans or the treatment in raising animals - cows, pigs, sheep, horses, where artificial insemination is performed. Finally, additional matter of invention constitute medicaments produced according to the application defined above.

The matter of invention is an application of effective quantity of barusiban for manufacture of medicaments applied in the procedures of assisted reproduction, specifically in the embryo transfer or artificial insemination. The medicaments defined in the invention are applied in form of barusiban before and/or during and/or after the embryo transfer as well as before and/or during and/or after the artificial insemination. These medicaments are administered enterally or parenterally in 24h dose from 0,01 mg up to 10g. The application of these medicaments relates to the treatment of infertility in humans especially for the in vitro fertilization and transfer of fresh embryos, or the transfer of frozen/thawed embryos, in the situation where both gametes (oocyte and sperm) are taken from partners of when one or both gametes are taken from the donor(s). Treatment with barusiban is carried out also in raising animals - cows, pigs, horses, sheep and covers the embryo transfer, where effective application is assumed in daily dose from 0,01mg up to 10g.

Treatment with barusiban relates to the procedure of artificial insemination in humans and in raising animals - cows, pigs, horses, sheep, where these are applied before and/or during and/or after the procedure. In such a treatment, medicaments that are defined in the invention are used for regulation of the uterine contractility, improving the transport of sperm in the female genital tract, when 24h dose from 0,01mg up to 10g is assumed.

Application of barusiban for manufacture of medicaments used in the procedures of assisted reproduction is carried out in combination with the application of one or more of substances of the following: nitric oxide donors, substrates of nitric oxide synthase, progestagens, prostaglandin antagonists, methyloxantines, beta agonists, prostacyclin agonists.

Good safety profile is one of favorable entities of medicaments containing antagonists of oxytocin, antagonists of oxytocin and vasopressin or antagonists of vasopressin, or their pharmaceutically accepted salts, for instance atosiban, barusiban or relcovaptan, especially because of high specificity and selectivity of these substances, usually limiting the action of these drugs to the uterus.

Inhibition of oxytocin and/or vasopressin receptors in the uterus results in improvement of uterine receptivity on several ways - firstly, by decrease of uterine contractility, secondly, thanks to beneficial influence on the state of endometrium that is reached by inhibition of local prostaglandin release (that release decreases endometrial perfusion). In case of uterine contractions directed from uterine fundus towards the cervix, inhibition of oxytocin and/or vasopressin receptors reached before, during or after the artificial insemination will prevent from expelling the injected sperm out from the uterus to vagina.

Combination of antagonists of oxytocin, antagonists of oxytocin and vasopressin or antagonists of vasopressin or their pharmaceutically accepted salts with other substances inhibiting uterine contractility, for instance beta agonists, exerts hyperadditive effect. Such an effect may be used for decreasing the doses of active substances in the combination medicaments, and it is connected to the enhancement of action of substances utilized in the composition. Consequently, it also decreases the probability of adverse drug reactions. Nitric oxide donors, nitric oxide synthase substrates, prostaglandins inhibitors, methyloxantines, prostacyclin mimetics or progestagens are examples of substances, which may be combined with antagonists of oxytocin or antagonists of oxytocin and vasopressin antagonists or antagonists of vasopressin or their pharmaceutically accepted salts, improving the uterine receptivity and regulating uterine contractility, and decreasing the likelihood for drug adverse reactions.

Invention will cause an increase of pregnancy rate after the embryo transfer and artificial insemination. In humans it constitutes a direct, social benefit for the population, additionally effecting in decrease of costs of infertility treatment. The application of an invention in raising animals will allow a decrease of costs of breed.

In contrast to the alternative application of oxytocin antagonists disclosed in patent WO 9609824 that is claimed to increase fertility and embryonic survival in farm animals, where it is achieved by the prolongation of function of corpus luteum, that provides the source of progesterone and supports the pregnancy, the present invention refers to the procedures of assisted reproduction not to spontaneous parturition. Adiditionally, present invention does not refer to the formation or maintenance of corpus luteum, as in cycles with embryo transfers no corpus luteum is present. Therefore, increase in pregnancy rates according to the invention is achieved on different mechanism of improvement of uterine receptivity, both in case of embryo transfers and artificial inseminations.

For better illustration of the invention, following figures have been attached.

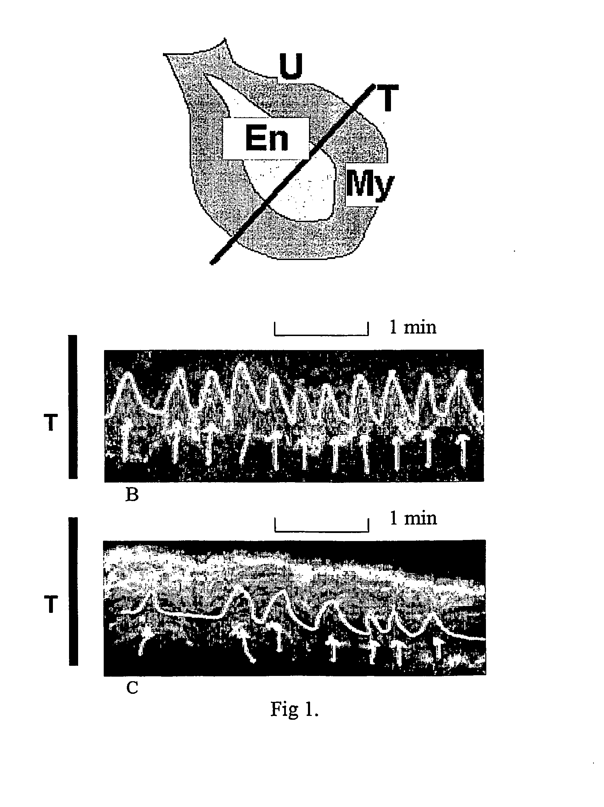

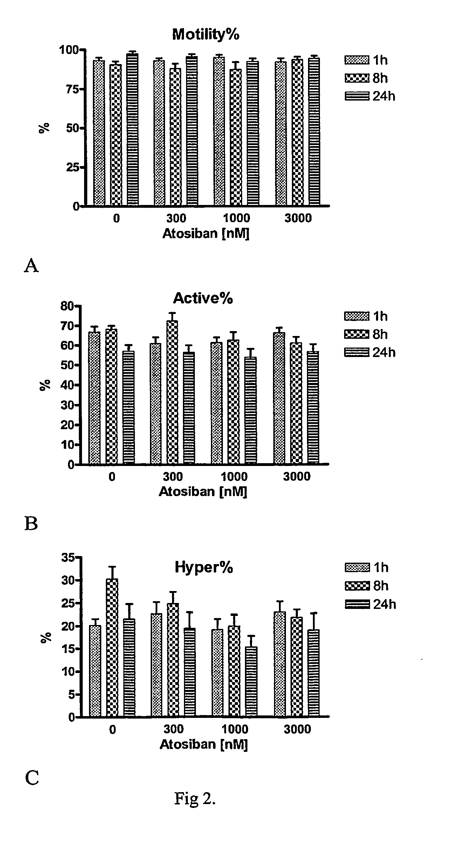

Fig 1 presents a graph on uterine contractile activity assessed by transvaginal sonography in patient which case is described in example 1. Uterine contractions on graph B and C are marked with light color for better visualization. In the method of digital image analysis, due to the requirement of a high picture quality, a period of 4 minutes out of total 5 minutes recorded was selected for analysis.
A) Schematic presentation of a principle of non invasive assessment of uterine contractility (done similarly to Fanchin et al. [Hum. Reprod. 1998;13(7):1968-74]) Description: U - uterus, En - endometrium (the inner layer of the uterus) - during the uterine contractions, changes in texture and contour of endometrium are observed, My - myometrium (the middle layer of uterus, built of smooth muscle tissue), T - testing segment, where movements of contour of endometrium representing the uterine contractile activity are assessed
B) Graph presenting uterine contractile activity before the application of oxytocin antagonist atosiban (reference). Contractile hyperactivity is seen. Legend: uterine contraction, 1 min - segment representing 1 min of digital recording, T - testing segment, where movements of contour of endometrium reflecting the uterine contractile activity are assessed; C) Graph presenting uterine contractile activity in the time of maximal effect of oxytocin antagonist atosiban. Uterine contractile activity is notably decreased. Legend: t-uterine contraction, 1 min - segment representing 1 min of digital recording, T - testing segment, where movements of contour of endometrium reflecting the uterine contractile activity are assessed
   Figure 2, constructed basing on example 2 presents a comparison of parameters of human sperm motility in control samples and in samples exposed to 3 concentrations of atosiban (reference) (300nM, 1000 nM i 3000 nM). Figure comprises of 3 graphs: A - graph presenting percentages of actively motile sperm in relation to the exposure to atosiban (0 nM, 300nM, 1000 nM i 3000 nM) and the time of exposure. B - graph presenting percentages of motile sperm in relation to the exposure to atosiban (0 nM, 300nM, 1000 nM i 3000 nM) and the time of exposure. C - graph presenting percentages of hyperactively motile sperm in relation to the exposure to atosiban (0 nM, 300nM, 1000 nM i 3000 nM) and the time of exposure. No significant differences between the control (0 nM) group and experimental (atosiban 300nM, 1000 nM lub 3000 nM) groups were observed. Decrease of sperm motility with time (in 8th and 24th hour of experiment) depended on gradual decrease of energetic reserve of sperm cells and was comparable in all assessed groups. In the statistic analysis (analysis of variance - ANOVA), no significant differences in the parameters of sperm motility were found. Legend: Active% - percentage of actively motile sperm cells; Motility% - percentage of motile sperm cells; Hyper% - percentage of hyperactively motile sperm cells.

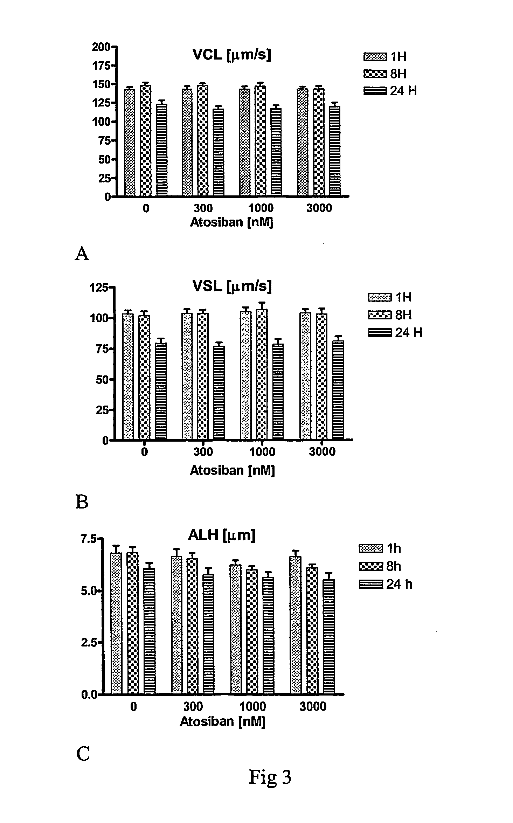

Figure 3, constructed basing on example 2 presents comparison of parameters of movement (velocity) of sperm cells in control samples and in samples of experimental groups (exposed to 3 concentrations of atosiban: 300nM, 1000 nM i 3000 nM). Figure 3 comprises of 3 graphs: A - graph presenting changes in total sperm velocity in relation to the exposure to atosiban (reference) (0nM, 300nM, 1000 nM i 3000 nM) and the time of exposure; B - graph presenting changes in straight-line sperm velocity in relation to the exposure to atosiban (0 nM, 300nM, 1000 nM i 3000 nM) and the time of exposure; C - graph presenting changes in amplitude of lateral head displacements of sperm cells in relation to the exposure to atosiban (0 nM, 300nM, 1000 nM i 3000 nM) and the time of exposure; Legend: VSL - straight line velocity, VCL - total velocity, ALH - amplitude of lateral head displacements. In the statistic analysis (analysis of variance - ANOVA), no significant differences between the control (onM) and experimental samples (atosiban 300nM, 1000 nM, 3000 nM) were found.

The results of the human sperm motility bioassay study, presented at Fig. 2 and Fig. 3, further referred in the example 2 confirms, that antagonists of oxytocin and vasopressin-atosiban (reference) does not inhibit human sperm motility in vitro, which confirms that it lacks embryotoxic potential, which mandates its application in assisted reproduction.

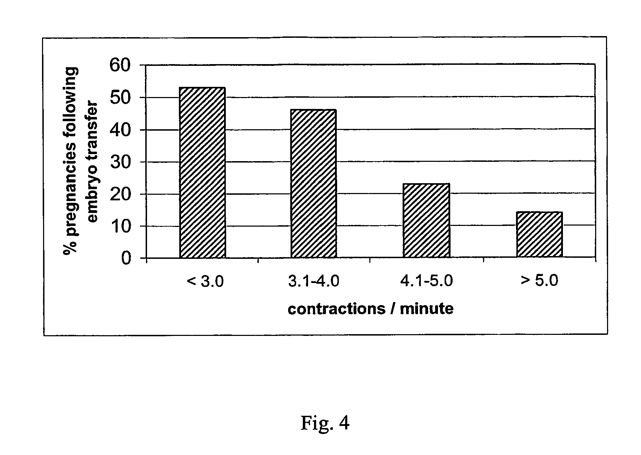

Fig 4 illustrates the relation between uterine contractile activity and embryo transfer success rates. In cases of high contractile activity (>5.0 contractions/minute), clinical pregnancy rate reached 14% and in case of patients of low uterine contractile activity it was over 3-fold increased, reaching 53% (figure according to publication of Fanchin et al [Hum. Reprod. 1998;13(7):1968-74])

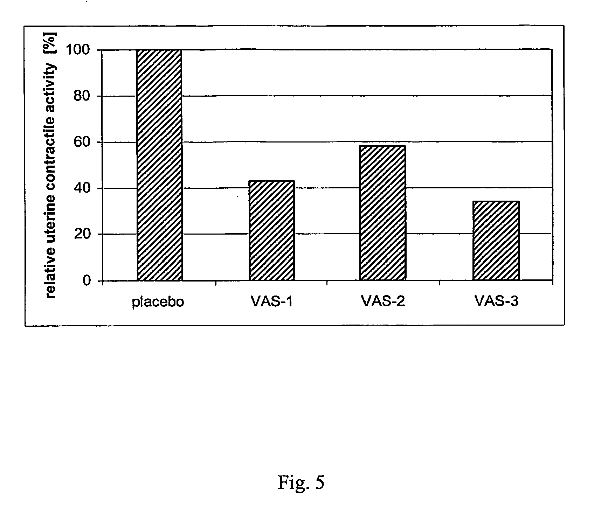

Figure 5 presents a graph on uterine contractile activity of non pregnant humans (assessed using the parameter of area under curve of intrauterine pressure) during the vasopressin stimulation in cases of absence and presence of oxytocin and vasopressin antagonist relcovaptan (reference) in the organism.

### Description of bars:

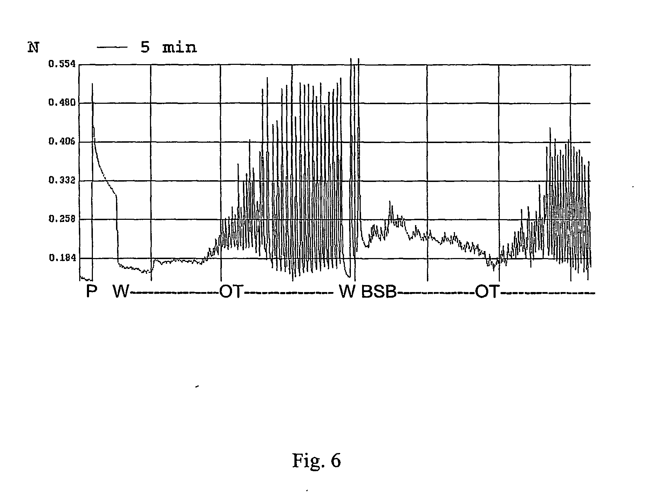

placebo - reference uterine contractile activity (in patients receiving placebo); VAS-1, VAS-2, VAS-3 - relative (to placebo) uterine contractile activity in patients given relcovaptan: respective numbers correspond to respective vasopressin administrations. Data presented at the figure show the effect of inhibition of vasopressin and oxytocin receptors (both vasopressin and relcovaptan act on oxytocin receptors as well), decreasing the uterine contractility. Considering receptor profile of relcovaptan (reference). greater affinity towards vasopressin V₁ₐ receptors as compared to oxytocin receptors, together with the fact that V₁ₐ expression is greater in non pregnant uteri as compared to pregnant, relcovaptan is a promising candidate for application before the embryo transfer and artificial insemination (figure according to publication ofBossmar et al. [BJOG 1997, 104; 471-477]) Figure 6 (according to Pierzynski P et al. [JSGE 2004, 11(6): 384-387]) illustrates the contractile activity of human pregnant myometrium before and after the administration of oxytocin antagonists barusiban
Legend P - reference potassium chloride (KCl) contraction W- washing
OT - series of cumulative oxytocin injections for stimulation of uterine contractility (repeated before and after administration of oxytocin antagonist), W - washing, BSB - administration of oxytocin antagonist barusiban
In this in vitro study an effect of barusiban on oxytocin stimulated uterine smooth muscle contractility was assessed. Uterine smooth muscle (myometrium) contractile activity was significantly reduced after the administration of barusiban, and total inhibition of contractile activity was observed in the range of oxytocin concentrations, that occur in humans. In this study it was shown that barusiban is more potent inhibitor of uterine contractions as compared to atosiban (reference). In spite of physiological differences between pregnant and non pregnant uteri barusiban is a promising candidate for application in assisted reproduction techniques considering its prolonged mode of action (estimated half time of a single dose - 7 hours).

The invention relates to the application of barusiban for manufacture of medicaments, containing active substances in the 24h dose between 0,01 mg and 10 g. Application of these medicaments is to improve the uterine receptivity, which is achieved by alleviation of the influence of oxytocin and/or vasopressin on the myometrium and endometrium (smooth muscle and innermost layer of uterus, where the implantation takes place). Such an effect causes uterine quiescence (in terms of contractility) and additionally ceases the stimulation of synthesis of prostaglandins in uterus (prostaglandins normally increase the uterine contractility and decrease endometrial perfusion). Consequently, intrauterine environment becomes more "embryo-friendly", which in turn increases the embryo transfer success rate. Medicaments described in the invention, when administered in cases of uterine contractions directed from the uterine fundus towards the uterine cervix, procedure, by preventing of expulsion of sperm out of the uterus towards the cervix.

The invention is useful for treatment improving the uterine receptivity at the embryo transfer in humans and raising animals. Treatment with antagonists of oxytocin, antagonists of oxytocin and vasopressin or antagonists of vasopressin or their pharmaceutically accepted salts may utilize peptide drugs, that follow the structure of oxytocin and vasopressin, namely barusiban . Peptide drugs may not be administered orally due to their digestion, such an administration may be however possible when special systems for drug release are applied. Peptide antagonists of oxytocin, oxytocin and vasopressin or antagonists of vasopressin, namely barusiban, or their pharmaceutically accepted salts may be administered intravenously, intramuscularly, subcutaneously or nasally. For reference: Non peptide antagonists of oxytocin, oxytocin and vasopressin or antagonists of vasopressin, for instance relcovaptan, or their pharmaceutically accepted salts may be administered also enterally. Medicaments described in the invention, containing barusiban will be dosed from 0,01 mg to 10g per 24h in single dose, repeated dose or as an continuous/intermittent infusion. Drug administration will start maximally one week before the embryo transfer and will last maximally until one week after the transfer. It will allow to abolish the uterine contraction reflex reaction to introducing the transfer catheter, as well as decrease the uterine contractility and improve the uterine receptivity until the implantation of an embryo, that in humans takes place about one week after the transfer.

Another aspect of the invention relates to manufacture of medicaments applied for regulation of uterine contractility before, during and after the artificial insemination. Barusiban will be administered in cases of uterine contractility directed from the uterine fundus towards the cervix, that may expel sperm from the uterus towards cervix. Application of medicaments described in the invention will start maximally a week before the insemination and will be continued maximally until one week after the procedure. In case of a series of inseminations, the treatment may be continued maximally until a week after the last insemination. Barusiban will be dosed from 0,01 mg to 10 g per 24h enterally or nasally, intravenously, intramuscularly or subcutaneously. Examples of forms for administration of these medicaments are tablets, dragettes, capsules, pills, suspensions, sirups, granulates and ' solutions. Every dosing unit, for instance a tablet or a spoon of solution may contain for instance 0,1-1000 mg of each of active constituents.

Medicaments containing barusiban may be administered in combination with other drugs, that may decrease the uterine contractility, for instance with nitric oxide donors, nitric oxide synthase substrates, progestagens, antagonists of prostaglandins, methyloxantines, beta agonists, prostacyclin agonists or progestagens.

Combinations of barusiban with other mentioned above substances may be administered enterally or parenterally in doses of appropriate efficacy in decreasing the uterine contractility with possible additional effect of reduction of adverse drug reactions.

Example 1 Clinical application of medicament containing atosiban - an antagonist of oxytocin and vasopressin for increasing the uterine receptivity at the embryo transfer in the course of assisted reproduction procedure of IVF-ET (in vitro fertilization-embryo transfer). (for reference)

Clinical description: 42 years old female with 15-years history of infertility, never pregnant, with negative obstetric/gynecologic history referred to the infertility clinic for the treatment. Initial, hormonal lab results done during the menses were as follows: FSH - 5,1 IU/ml (normal range 3,2 - 10,0), LH - 1,8 mIU/ml (normal range 1,2-12,5), PRL - 23,4 ng/ml (normal range 3,3- 24,5), E₂ 95,7 pg/ml (normal range 12,0-48,0), T - 0,43 (normal range 0,1-0,96ng1m1). On physical and sonographic evaluation, myomatous uterus and left ovarian cyst were demonstrated. Patient was treated surgically one month after the initial visit by a laparotomy and enucleation of uterine myomata and enucleation of left ovarian cyst, with no complications.

7 months after the operation, specific treatment of infertility was initiated, and patient was included within the IVF-ET program. The first program of treatment was done in September 2003, applying long protocol of stimulation of ovulation. In the course of this treatment patient received in total 44 ampoules of recombinant FSH (Gonal 75 IU) and 8 of menopausal gonadotropin HMG (Menogon 75 IU), which induced the growth of 3 Graaf follicles. After the transvaginal aspiration of follicles, 1 MII oocyte was collected. Due to the azoospermy found in patient's husband, further confirmed by testicular biopsy, in vitro fertilization utilized sperm of an anonymous donor. On the third day after the oocyte collection, one, 4-blastomere embryo was transferred into the uterus, with negative result.

Next (2^{nd}) IVF program was done 3 months after. In the course of controlled ovarian stimulation in total 74 ampoules of recombinant FSH (Gonal 75 IU) and 3 ampoules of human menopausal gonadotropin (Menogon 75 IU) were administered. In this cycle of treatment, a growth of 3 follicles was confirmed. Transvaginal follicle aspiration resulted in collection of 3 MII oocytes, which were consecutively fertilized with sperm of an anonymous donor. On the third day after the oocytes collection, two 2-blastomere embryos were transferred into the uterus, with negative result.

The third IVF program done after following 6 months also utilized long protocol of ovarian stimulation. During that cycle of treatment, 58 amp of recombinant FSH (Gonal 75 IU) and 2 amp of menopausal gonadotropin HMG (Menogon 75 IU) were administered. In result, a growth of 8 follicles was observed. After the transvaginal follicle aspiration, 2 MII oocytes were collected, which were consecutively fertilized with sperm of an anonymous donor. Two, 4-blastomere embryos were transferred into the uterine cavity, with negative result. One month later, hysteroscopy, aiming to provide data on possible pathology of uterine cavity was done, with negative result.

In view of the high probability of low developmental potential of patient's oocytes, after further 2 months, a decision on changing the mode of treatment to using donor's oocyte was taken. Oocytes were anonymously donated by another, healthy patient treated in the IVF program due to male infertility. After the in vitro fertilization of 2 donated oocytes with sperm of anonymous donor an embryo transfer was performed with negative result. Afterwards, throughout the following 7 months, 3 consecutive cycles of donor oocyte-donor sperm embryos were performed, with negative result.

In the 8^{th} cycle of the treatment, a decision on application of a medicament containing atosiban (antagonist of oxytocin and vasopressin) was drawn, aiming to improve the uterine receptivity. The medicament was given in intravenous infusion, which started 60 minutes before the transfer and was continued throughout 2 hours after the transfer. 10 minutes before the start of administration (70 minutes before the transfer) transvaginal sonography with 5-minute digital registration of uterine contractions was performed. 60 minutes before the transfer a bolus dose of 6,75mg of atosiban was given intravenously, with no adverse reactions. Immediately after, a continuous infusion of 18mg/hour of atosiban was connected using the infusion pump and was continued throughout the next 60 minutes. 45 minutes after the initiation of the infusion, transvaginal sonography with digital image registration was repeated. Between 55^{th} and 60^{th} minute of infusion, a sonography-guided transfer of 2 embryos was performed. After the transfer, the dosage of atosiban was changed to 6 mg/hour. Infusion was finished 2 hours after the transfer, total dose administered was 37,5 mg.

The mode of application of atosiban described above provided the maximal effect at the time of embryo transfer (it was proven that stable concentrations of atosiban are reached about an hour after the initiation of the infusion). Patient stayed in horizontal position until an hour after termination of the infusion and limited her activities on that day. At the control visit done 2 weeks after the embryo transfer, a pregnancy test was done with positive result. On the next visit (4 weeks after the transfer) intrauterine, normal twin pregnancy was confirmed by transvaginal sonography, fetal heart beats were visualized in both gestational sacs.

Positive result of the infertility treatment in that patient confirms the beneficial effect of antagonists of oxytocin and vasopressin on enhancing the uterine receptivity at the embryo transfer. Since it is not possible to confirm another aspects of action of atosiban (its effect on endometrial perfusion or on local prostaglandins production) during the transfer, the proof of action was based on its effect of decreasing of the uterine contractile activity, which was non-invasively demonstrated in the moment of maximal effect of atosiban. Analysis of uterine contractility was done by two methods, utilizing sonographic image sequences: by inspection and manual registration of contractions on an accelerated film sequence (method I), and by digital analysis of displacements of endometrial contour (method II, done similarly to the one presented by R. Fanchin et al [Fanchin R i wsp. Hum Reprod 1998;13(7):1968-74]. Analysis of the uterine contractile activity done in the situation of lack of action and in the situation of stable blood levels of atosiban demonstrated its significant reduction (method I: 15 contractions/5min before the administration of atosiban vs 8 contracitons/5min after the administration. Method II: 11 contractions/4 min before the administration of atosiban vs. 7 contracitons/4 min after the administration). Method II is illustrated at Figure 1. Application of a medicament containing atosiban improved the uterine receptivity on several ways: directly - by decreasing uterine contractile activity, as well as indirectly - by reducing the stimulation of uterine contractility and alleviating the effect of decrease of the endometrial perfusion, which resulted from the inhibition of production/excretion of prostaglandins in the uterine cavity.

In recapitulation, this case confirms the rationale for application of medicaments containing antagonists of oxytocin, antagonists of oxytocin and vasopressin or antagonists of vasopressin or their pharmaceutically accepted salts in the treatment improving the uterine receptivity in assisted reproduction, especially during the procedure of embryo transfer. Additionally, pregnancy was achieved in second patient with similar clinical characteristics.

### Example 2. Safety of oxytocin antagonists in the assisted reproduction (for reference)

Medicament containing atosiban is up to date the only one antagonists of oxytocin and vasopressin registered for use in humans. It is a peptide substance, registered for intravenous application in situation diametrically different from assisted reproduction - in preterm labor, where it is being used for prolonging the pregnancy. Clinical safety of atosiban was thoroughly verified in studies preceding the registration. Atosiban is considered uniquely safe, causing minimal side effects. However, no application in assisted reproduction was presumed as no embryotoxicity studies were done on atosiban.

In order to verify the embryotoxicity of atosiban a study on influence of atosiban on parameters of human sperm motility was performed (Human Sperm Motility Bioassay). The technique used in the study is a standard in the quality control and evaluation of embryotoxicity in IVF laboratories. It was shown, that this technique is comparable to the mouse or rabbit embryo bioassays, which are animal models for embryotoxicity [Miller, JW et al. Fertil Steril 2001: 76(3), Suppl 1;S104]. Specificity and sensitivity of Human Sperm Motility Bioassay is comparable to tests performed on embryos and currently it is the most popular due to lower cost and complexity. It was demonstrated that when a certain factor in the human sperm environment limits its motility, it will be embryotoxic as well.

The study was performed on samples of fresh human sperm taken from 15 healthy donors with perfect seminologic parameters. After selecting the alive and motile population (swim-up ascending migration method), sperm samples were transferred to the sterile Eppendorf tubes containing Human Sperm Preparation Medium alone or with atosiban in concentrations of 300nM, 1000nM, 3000nM, last concentrations being 10-fold to maximal concentrations achieved in humans in the course of treatment of preterm labor. Sperm motility assessment was performed with computer assisted sperm analyzer Hobson Sperm Tracker in the 1^{st}, 8^{th} and 24^{th} hour of exposition to atosiban.

Following parameters were analyzed:
a) percentage of actively motile sperm cells (Active%); b) percentage of motile sperm cells (Motility%); c) percentage of hyperactively motile sperm cells (Hyper%), d) VSL - straight line velocity, e) VCL - total velocity, f) ALH - amplitude of lateral head displacements. Results are presented at Figure 2 and Figure 3. In the statistic analysis, analysis of variance (ANOVA) was applied. No significant differences between the control samples and the ones exposed to atosiban was however found. It confirms lack of embryotoxicity of atosiban, which mandates its utilization in assisted reproduction.

## Claims

1. Use of barusiban for the manufacture of a medicament for an improved uterine receptivity in assisted reproduction.

2. Use according to claim 1, wherein the manufactured medicament is to be applied before, during and after the embryos transfer into the uterine cavity.

3. Use according to claim 1, wherein barusiban is to be applied in 24h dose from 0.01 up to 10 g.

4. Use according to claim 1 wherein the manufactured medicament is to be utilized for the treatment of infertility in humans or for the assisted reproduction in animals, that relates to embryo transfer and/or artificial insemination.

5. Use according to claim 4 wherein embryo transfer is the transfer of fresh or frozen/thawed embryos.

6. Use according to claim 1 wherein it relates to the procedures of in vitro fertilization-embryo transfer (IVF-ET).

7. Use according to claim 1 wherein it relates to the procedures of assisted reproduction in raising animals, where embryo transfer or artificial insemination is done.

8. Use according to claims 4 to 7, wherein raising animals are from the group as follows: cows, pigs, sheep and horses.

9. Use according to claim 1 wherein barusiban is to be administered in combination with the application of other medicaments for the treatment in assisted reproduction from the group as follows: nitric oxide donors, nitric oxide synthase substrates, progestagens, prostaglandin antagonists, methyloxanthines, beta agonists, prostacyclin agonists.

## Patentansprüche

1. Verwendung von Barusiban zur Herstellung eines Arzneimittels für eine verbesserte Gebärmutter-Aufnahmefähigkeit bei der künstlichen Fortpflanzung.

2. Verwendung gemäss Anspruch 1, wobei das hergestellte Arzneimittel vor, während und nach dem Embryotransfer in die Gebärmutterhöhle angewendet werden soll.

3. Verwendung gemäss Anspruch 1, wobei Barusiban in einer 24 Stunden-Dosis von 0,01 bis zu 10 g angewendet werden soll.

4. Verwendung gemäss Anspruch 1, wobei das hergestellte Arzneimittel für die Behandlung von Unfruchtbarkeit in Menschen oder für die assistierte Fortpflanzung in Tieren verwendet werden soll, die sich auf Embryotransfer und/oder künstliche Besamung bezieht.

5. Verwendung gemäss Anspruch 4, wobei der Embryotransfer der Transfer von frischen oder gefrorenen/aufgetauten Embryonen ist.

6. Verwendung gemäss Anspruch 1, wobei diese sich auf die Verfahren in vitro-Befruchtung-Embryotransfer (IVF-ET) bezieht.

7. Verwendung gemäss Anspruch 1, wobei diese sich auf die Verfahren der assistierten Fortpflanzung in Aufzuchttieren bezieht, wobei ein Embryotransfer oder künstliche Besamung vorgenommen wird.

8. Verwendung gemäss Ansprüchen 4 bis 7, wobei die Aufzuchttiere aus folgender Gruppe sind: Kühe, Schweine, Schafe und Pferde.

9. Verwendung gemäss Anspruch 1, wobei Barusiban in Kombination mit der Anwendung anderer Arzneimittel für die Behandlung bei der assistierten Fortpflanzung aus der folgenden Gruppe: Stickoxid-Donoren, Stickoxid-Synthasesubstraten, Progestagenen, Prostaglandin-Antagonisten, Methyloxanthinen, β-Agonisten, Prostacyclin-Agonisten verabreicht werden soll.

## Revendications

1. Utilisation de barusiban pour la fabrication d'un médicament en vue d'avoir une meilleure réceptivité utérine lors d'une reproduction assistée.

2. Utilisation selon la revendication 1, dans laquelle le médicament fabriqué doit être appliqué avant, pendant et après le transfert d'embryons dans la cavité utérine.

3. Utilisation selon la revendication 1, dans laquelle le barusiban doit être appliqué en une dose de 24h de 0,01 à 10 g.

4. Utilisation selon la revendication 1, dans laquelle le médicament fabriqué doit être utilisé pour le traitement de l'infertilité chez les humains ou pour la reproduction assistée chez les animaux, qui concerne un transfert d'embryons et/ou une insémination artificielle.

5. Utilisation selon la revendication 4, dans laquelle le transfert d'embryons est le transfert d'embryons frais ou congelés/décongelés.

6. Utilisation selon la revendication 1, où elle concerne les procédures de transfert d'embryons obtenus par fécondation in-vitro (FIVETE).

7. Utilisation selon la revendication 1, où elle concerne les procédures de la reproduction assistée dans l'élevage des animaux, où le transfert d'embryons ou l'insémination artificielle sont exécutés.

8. Utilisation selon les revendications 4 à 7, dans laquelle l'élevage des animaux est constitué du groupe suivant : vaches, cochons, moutons et chevaux.

9. Utilisation selon la revendication 1 dans laquelle le barusiban doit être administré en combinaison avec l'application d'autres médicaments pour le traitement lors d'une reproduction assistée du groupe suivant : donneurs d'oxyde nitrique, substrats pour l'oxyde nitrique synthéase, progestagènes, antagonistes de la prostaglandine, méthyloxanthines, béta-agonistes, agonistes de la prostacycline.
